Europäisches Patentamt

European Patent Office

Office européen des brevets

(11) Numéro de publication: **0 301 968 B1**

(12) **FASCICULE DE BREVET EUROPEEN**

(45) Date de publication de fascicule du brevet: **04.11.92** (51) Int. Cl.⁵: **C12Q 1/70**

(21) Numéro de dépôt: **88401964.7**

(22) Date de dépôt: **28.07.88**

(54) **Sondes d'acides nucléiques des virus de papillome humain.**

(30) Priorité: **31.07.87 FR 8710917**
**05.04.88 FR 8804436**

(43) Date de publication de la demande:
**01.02.89 Bulletin 89/05**

(45) Mention de la délivrance du brevet:
**04.11.92 Bulletin 92/45**

(84) Etats contractants désignés:
**AT BE CH DE ES FR GB GR IT LI LU NL SE**

(56) Documents cités:
EP-A- 0 133 123      EP-A- 0 192 001
EP-A- 0 200 362      EP-A- 0 235 004
EP-A- 0 243 221      WO-A-83/03623
WO-A-86/05816

CHEMICAL ABSTRACTS, vol. 107, 1987, Columbus, OH (US); S.SYRJANEN et al., p. 409, no. 232380e

CHEMICAL ABSTRACTS, vol. 105, 1986, Columbus, OH (US); K.MILDE et al., p. 527, no. 169878q

CHEMICAL ABSTRACTS, vol. 104, 14 avril 1986, Columbus, OH (US); J.K.McDOUGALL et al., p. 340, no. 125877p

(73) Titulaire: **LA REGION WALLONNE**
**7 Avenue du Prince de Liège**
**B-5100 Namur(BE)**

(72) Inventeur: **Herzog, Albert**
**Elewijtsesteenweg 190**
**B-1840 Eppegem(BE)**
Inventeur: **Cravador, Alfredo**
**Avenue des Cygnes 21**
**B-1640 Rhose-Saint-Genese(BE)**
Inventeur: **Houard, Sophie**
**Avenue Seghers 28**
**B-1080 Bruxelles(BE)**
Inventeur: **Bollen, Alex**
**Gaasbeekstraat 65**
**B-1711 Itterbeek(BE)**

(74) Mandataire: **Ahner, Francis et al**
**CABINET REGIMBEAU, 26, avenue Kléber**
**F-75116 Paris(FR)**

Rank Xerox (UK) Business Services

**Description**

Le domaine technique de la présente invention est celui des sondes constituées par des séquences d'acides nucléiques, ADN mono-brin ou ARN, marquées.

De telles sondes sont bien connues dans l'état de la technique et peuvent être obtenues par diverses voies, notamment par génie génétique ou par synthèse directe manuelle ou automatique.

Ces séquences d'acides nucléiques ont la propriété de s'apparier et de former des hybrides avec les séquences complémentaires d'ADN, le cas échéant préalablement dénaturé, si ceux-ci étaient initialement bicaténaires, ou d'ARNm. Cette dénaturation peut se faire après incubation dans un milieu de haute force ionique et à température élevée ou dans un milieu basique. Ces hybrides sont ensuite détectables.

La détection des hybrides peut se faire par différentes méthodes. La sonde peut être marquée par l'un des procédés connus pour le marquage des sondes d'acides nucléiques. Il peut s'agir d'un marquage radioactif, par exemple avec le phosphore 32, ou bien dans le cas d'une sonde froide d'un marquage non radioactif, par exemple, enzymatique, comme cela est également connu. Dans certains cas, la sonde n'est pas marquée lors de son utilisation proprement dite, mais modifiée chimiquement pour pouvoir être repérée après l'hybridation, par exemple avec la biotine.

Plus précisément, la présente invention concerne des sondes d'acides nucléiques permettant de détecter les différents types de virus de papillomes humains par hybridation avec l'ADN du virus. Il s'agit, notamment, des huit types de virus de papillomes humains (HPV) dont les totalités des séquences ADN sont à ce jour connues, à savoir les types HPV1a, HPV5, HPV6b, HPV8, HPV11, HPV16, HPV18 et HPV33.

10 % des femmes sont infectées par le virus HPV ; le virus se localise dans les cellules cervicales et conduit dans un certain nombre de cas, à des lésions dysplasiques et/ou malignes du col utérin. Parmi les nombreux types de virus HPV, HPV16 et HPV18 semblent le plus fréquemment associés à ces lésions.

Les HPV se caractérisent par un génome à ADN double brin circulaire, long d'environ 8000 paires de bases, enveloppé dans une capside protéique de 60 nm de diamètre. Le virus subsiste dans les lésions non malignes à l'état d'épisome non intégré. On observe dans ces cas la perturbation de la différentiation cellulaire et la production de particules virales aux stades tardifs de la différentiation. Lorsque le carcinome utérin est établi, on constate une intégration variable de certaines séquences de l'ADN viral dans l'ADN cellulaire.

La détection des virus HPV dans le col utérin s'effectuait par l'emploi de sondes moléculaires constituées de fragments d'ADN viral clonés et marqués isotopiquement.

Dans le cadre d'études épidémiologiques et du diagnostic en routine des infections par HPV, il est utile de développer une méthode rapide, simple, spécifique et sensible basée sur l'emploi de sondes à ADN synthétique marquées de façon non isotopique et utilisables in situ. A cet effet, on a identifié, au niveau de l'ADN, des régions spécifiques des différents types de virus $\overline{\overline{HPV}}$ pour en déduire des sondes synthétiques homologues complémentaires. Les séquences nucléotidiques de HPV1, HPV5, HPV6, HPV11, HPV16, HPV18 et HPV33 sont connues.

Un but de la présente invention est de proposer des sondes caractéristiques de l'ensemble des différents types de virus HPV notamment cités ci-dessus qui soient efficaces, c'est-à-dire des sondes présentant des séquences d'acides nucléiques les plus longues possibles et qui soient communes aux différents types de virus HPV.

Un autre but de la présente invention est également de proposer des sondes spécifiques de chacun des différents types de virus HPV et qui assurent un appariement stable.

La présente invention a donc pour objet une sonde d'acides nucléiques utile pour détecter indifféremment les différents types de virus de papillomes humains, notamment HPV1a, HPV5, HPV6b, HPV8, HPV11, HPV16, HPV18 et HPV33, caractérisée en ce qu'elle comporte l'oligomère :

X-A-A-A-A-C-G-A-A-A-G-X

ou son complémentaire en interchangeant A et X d'une part, C et G d'autre part, dans le cas où l'ADN cible du virus était initialement bicaténaire et a été dénaturé avant d'être sondé, avec X = T ou U dans le cas où l'on sonde de l'ARNm.

A, T, C, G, U représentent les nucléotides correspondant aux bases adénine, thymine, cytosine, guanine et uracile respectivement.

La séquence T-A-A-A-A-C-G-A-A-A-G-T est la seule qui se retrouve en effet, notamment, dans l'ADN de chacun des huit types de virus HPV parmi des séquences d'au moins 10 bases de long.

Cette séquence correspond aux séquences situées sur le brin codant aux positions du premier nucléotide suivantes dans les différents types de virus :

2

pour HPV1a : position 1029 à 1040
pour HPV5 : position 1190 à 1201
pour HPV6b : position 1076 à 1087
pour HPV8 : position 1174 à 1185
pour HPV11 : position 1076 à 1087
pour HPV16 : position 1121 à 1132
pour HPV18 : position 1167 à 1178
pour HPV33 : position 1132 à 1143

La présente invention a également pour objet une sonde d'acides nucléiques commune aux différents types de virus HPV, caractérisée en ce qu'elle comprend le mélange de six oligomères comportant chacun la séquence commune de douze nucléotides visée précédemment, prolongée en amont comme en aval par les nucléotides correspondant aux huit types de virus HPV. Ces prolongements sont constitués par les nucléotides s'appariant aux nucléotides entourant immédiatement la séquence commune aux huit types de virus HPV T-A-A-A-C-G-A-A-A-G-T. Parmi ces oligomères, quatre sont associés chacun à un type de virus différent, le cinquième est commun aux virus 6b et 11, un autre est commun aux virus 5 et 8.

Par exemple, on peut prolonger la séquence commune de douze nucléotides de part et d'autre de l à 10 des nucléotides correspondants pour chaque type de virus.

En prolongeant jusqu'à six ou sept nucléotides de part et d'autre de la séquence commune, on obtient les six oligomères suivants ou leurs complémentaires :

pour HPV1a   A-X-G-C-X-X-X-A-A-A-A-C-G-A-A-AG-X-X-A-C-X-X-X

pour HPV6b et HPV11   A-G-GA-C-C-X-A-A-A-A-C-G-A-A-A-G-X-A-X-X-X-A-G

pour HPV8 et HPV5   C-A-A-A-A-A-C-X-A-A-A-A-C-G-A-A-A-G-X-A-X-C-X-X-A

pour HPV16   A-G-G-X-X-C-X-A-A-A-A-C-G-A-A-A-G-X-A-X-X-X-G-G

pour HPV18   A-X-G-X-X-X-X-A-A-A-A-C-G-A-A-A-G-X-X-X-G-C-A-G

pour HPV33   G-X-G-C-A-C-X-A-A-A-A-C-G-A-A-A-G-X-X-X-G-C-C-G

Ces oligomères correspondent à des séquences cibles d'ADN des virus aux positions suivantes pour chaque type de virus HPV :

pour HPV1a : position 1023 à 1047
pour HPV5 : position 1183 à 1207
pour HPV6b : position 1070 à 1093
pour HPV8 : position 1167 à 1191
pour HPV11 : position 1070 à 1093
pour HPV16 : position 1115 à 1138
pour HPV18 : position 1161 à 1184
pour HPV33 : position 1126 à 1149

Un prolongement de quatre bases de chaque côté de la séquence commune ne fournit que des séquences donnant des températures de demi-hybridation de l'ordre de 50 à 52°C. En revanche, à partir de six bases, tant vers l'amont que vers l'aval, correspondant donc aux séquences exactes présentes dans les virus, on obtient des températures de demi-hybridation de l'ordre de 60 à 70°C.

Ainsi, pour la séquence cible de HPY1a (en position 1023) la sonde a une température de demi-hybridation prévisible de 60°C,

pour HPV5 (position 1183) T = 62°C
pour HPV6b (position 1070) T = 64°C
pour HPV8 (position 1167) T = 62°C
pour HPV11 (position 1070) T = 64°C
pour HPV16 (position 1115) T = 64°C
pour HPV18 (position 1161) T = 62°C
pour HPV33 (position 1126) T = 70°C

Ces sondes oligonucléotidiques communes à tous les virus de papillomes humains connus permettent le diagnostic rapide des affections à base de virus de papillome.

Un autre objet de la présente invention est également une série de huit sondes d'acides nucléiques chacune spécifique d'un type de virus de papillome humain par rapport aux autres types, à savoir les oligomères suivants ou leurs complémentaires :

pour HPV1a : l'oligomère C-C-X-C-A-G-G-C-G-A-G-A-A-G-C-G-C-G-G-A-C

pour HPV5 : l'oligomère X-G-X-A-X-G-G-G-A-X-C-G-G-G-G-G-C-G-X-X-X-A

pour HPV6b : l'oligomère C-A-X-X-A-A-C-G-C-A-G-G-G-C-G-C-C-X-G-A-A

pour HPV8 : l'oligomère A-C-A-C-C-G-C-C-G-C-C-A-A-G-A-C-C-C-C-A

pour HPV11 : l'oligomère G-G-C-G-X-G-X-C-G-G-C-G-C-C-G-C-C-X-A-G

pour HPV16 : l'oligomère C-A-G-A-A-C-C-G-G-A-C-A-G-A-G-C-C-C-A-X

pour HPV18 : l'oligomère C-G-G-X-A-X-C-C-G-C-X-A-C-X-C-A-G-C-X-X-G

pour HPV33 : l'oligomère C-G-X-C-C-X-C-C-G-G-X-X-A-C-X-G-X-A-G-A-C-A

avec X = T ou U.

On peut envisager d'utiliser les oligomères complémentaires lorsque l'ADN à sonder était initialement bicaténaire et a été dénaturé pour pouvoir s'hybrider ou dans le cas où l'on sonde de l'ARNm.

L'oligomère sélectif pour HPV1a comprend 21 monomères, cette séquence correspond à la position 1052 à 1072 de l'ADN du virus et présente une température de demi-hybridation de 72°C.

L'oligomère sélectif pour HPV5 comprend 22 monomères, cette séquence correspond à la position 1801 à 1822 de l'ADN du virus et présente une température de demi-hybridation de 68°C.

L'oligomère sélectif pour HPV6b comprend 22 monomères, cette séquence correspond à la position 4761 à 4782 de l'ADN du virus et présente une température de demi-hybridation de 70°C.

L'oligomère sélectif pour HPV8 comprend 21 monomères, cette séquence correspond à la position 3344 à 3364 de l'ADN du virus et présente une température de demi-hybridation de 72°C.

L'oligomère sélectif pour HPV11 comprend 20 monomères, cette séquence correspond à la position 3416 à 3435 de l'ADN du virus et présente une température de demi-hybridation de 72°C.

L'oligomère sélectif pour HPV16 comprend 20 monomères, cette séquence correspond à la position 695 à 714 de l'ADN du virus et présente une température de demi-hybridation de 64°C.

L'oligomère sélectif pour HPV18 comprend 21 monomères, cette séquence correspond à la position 3448 à 3468 de l'ADN du virus et présente une température de demi-hybridation de 64°C.

L'oligomère sélectif pour HPV33 comprend 22 monomères, cette séquence correspond à la position 4474 à 4495 de l'ADN du virus et présente une température de demi-hybridation de 68°C.

La finalité de cette seconde série de sondes spécifiques de l'un ou l'autre des huits virus de papillomes humains est d'être utilisée comme éléments sélectifs pour des tests diagnostics d'infections par ces virus.

La présente invention a encore pour objet une seconde série de huit sondes d'acides nucléiques d'ADN mono-brin ou d'ARN, chacune spécifique d'un type de virus de papillome humain par rapport aux autres types. Il s'agit de sondes sélectives alternatives par rapport à celles décrites précédemment.

Ces sondes comportent une séquence d'acides nucléiques marquées comprenant les oligomères suivants :

pour HPV1a : CXGXCCCXXCXGCGCGAGACGX

pour HPV5 : XXGXAXGGGAXCGGGGGCGXXXA

pour HPV6b : GGCAAXCAXXAACGCAGGGGCG

pour HPV8 : GCAGCXCAGXCCGCGGCXCC

pour HPV11 : GAAGCXCXCCCAAGCCXGCXAXX

4

pour HPV16 : ACCAGAGACAACXGAXCXCXACXG

et : ACAAGCAGAACCGGACAGAGCCCA

pour HPV18 : CCCAXXGXAXCACCCACGGCCC

pour PHV33 : GXCCXCCGGXXACXGXAGACACX

Les séquences ci-dessus correspondent aux séquences de la fibre ou brin direct(e) ou codant(e) de l'ADN du virus et peuvent s'hybrider avec l'ADN bicaténaire ou avec la fibre inverse lorsque l'ADN a été dénaturé.

On peut aussi, bien entendu, utiliser des oligomères inverses complémentaires en interchangeant A et X d'une part, et C et G d'autre part. Ces oligomères inverses complémentaires sont notamment utiles comme sondes pour s'hybrider avec l'ARN messager du virus.

On rappelle que dans les séquences ci-dessus, A,T,C,G et U représente les nucléotides correspondant aux bases adénine, thymine, cytosine, guanine et uracyl respectivement, et X représente T ou U.

L'oligomère sélectif pour HPV1a comprend 22 monomères. Le premier nucléotide de cette séquence correspond à la position 3448 du brin codant de l'ADN du virus. La séquence se termine à la position 3469. Cette séquence correspond à une position située dans le gène E3 du virus HPV1a et présente une température de demi-hybridation de 72°C.

L'oligomère sélectif pour HPV5 comprend 23 monomères. Cette séquence correspond à la position 1800-1822 de l'ADN du virus située dans le gène E1 et présente une température de demi-hybridation de 70°C.

L'oligomère sélectif pour HPV6b comprend 22 monomères. Cette séquence correspond à la position 4755-4776 de l'ADN du virus située dans le gène L2 et présente une température de demi-hybridation de 70°C.

L'oligomère sélectif pour HPV8 comprend 20 monomères. Cette séquence correspond à la position 1208-1227 de l'ADN du virus située dans le gène E1 et présente une température de demi-hybridation de 70°C.

L'oligomère sélectif pour HPV11 comprend 23 monomères. Cette séquence correspond à la position 4637-4659 de l'ADN du virus située dans le gène L2 et présente une température de demi-hybridation de 70°C.

Le premier oligomère sélectif pour HPV16 comprend 24 monomères. Cette séquence correspond à la position 609-632 de l'ADN du virus située dans le gène E7 et présente une température de demi-hybrydation à 70°.

Pour le virus HPV16 une seconde sonde spécifique de 24 monomères a été déterminée, elle est située dans le gène E7 dépourvu de toutes bases T ou U, elle est repérée en position 690-713.

L'oligomère sélectif pour HPV18 comprend 22 monomères. Cette séquence correspond à la position 5486-5507 de l'ADN du virus située dans le gène L2 et présente une température de demi-hybridation de 72°C.

L'oligomère sélectif pour HPV33 comprend 23 monomères. Cette séquence correspond à la position 4475-4497 de l'ADN du virus située dans le gène L2 et présente une température de demi-hybridation de 70°C.

La présente invention a également pour objet une autre série de sondes spécifiques de chacun des types de virus HPV par rapport aux autres types de virus. Ces sondes présentent une longueur de 35 monomères. Il s'agit des sondes spécifiques précédentes, rallongées vers l'amont ou vers l'aval selon les zones d'analogie repérées. Ces oligomères correspondent également à la fibre codante de l'ADN du virus. L'invention a donc également pour objet les séquences complémentaires qui peuvent notamment s'hybrider avec l'ARN messager.

Ces nouvelles sondes spécifiques consistent en des séquences d'acides nucléiques marquées, comportant les oligomères suivants :

pour HPV1a : XGXCCCXXCXGCGCGAGACGXXGGAAGXAXACACA.

Cette séquence correspond à la position 3449-3482 du brin codant de l'ADN du virus et est située dans le gène E3.

Pour HPV5 : XGXAXGGGAXCGGGGGCGXXXAGCCAXGGACCAXA.

Cette séquence correspond à la position 1801-1835 de l'ADN du virus, elle est située dans le gène E1.

Pour HPV6b : GCAAXCAXXAACGCAGGGGCGCCXGAAAXXGXGCC.

Cette séquence correspond à la position 4756-4790 de l'ADN du virus et est située dans le gène L2.

Pour HPV8 : GCAGCXCAGXCCGCGGCXCCAGXCAAXAXCACXGX.

Cette séquence correspond à la position 1208-1242 de l'ADN du virus et est située dans le gène E1.

Pour HPV11 : XAXAXACCCXXGGGAAGCXCXCCCAAGCCXGCXAX.

Cette séquence correspond à la position 4624-4658 de l'ADN du virus et est située dans le gène L2.

Pour HPV16 : XGXXAGAXXXGCAACCAGAGACAACXGAXCXCXAC.

Cette séquence correspond à la position 596-630 de l'ADN du virus et est située dans le gène E7.

Pour HPV18 : CCCAXXGXAXCACCCACGGCCCCXGCCXCXACACA.

Cette séquence correspond à la position 5486-5520 de l'ADN du virus et est située dans le gène L2.

Pour HPV33 : XCCXCCGGXXACXGXAGACACXGXXGGACCXXXAG.

Cette séquence correspond à la position 4476-4510 de l'ADN du virus et est située dans le gène L2.

La présente invention a également pour objet d'autres sondes d'acides nucléiques spécifiques de chacun des types de virus de papillome humain, mais toujours situées dans la région E7 du génome de chacun de ces virus.

En effet, le gène E7 du génome de chacun de ces virus HPV est le plus concerné dans la forme intégrée de ces virus dans l'ADN des cellules malignes.

Cette troisième série de sondes spécifiques comporte des oligonucléotides d'une longueur telle qu'ils présentent une température de demi-hybridation de 70°C. Il s'agit des oligomères suivants ou de leurs complémentaires:

| Virus | Oligomère | Longueur | Position | Gène |
|-------|-----------|----------|----------|------|
| HPV1a | GACCGXCCXCGCGGAXCACAG | 21 | 711-731 | E7 |
| HPV5 | GCCXGACAACGAAAGGAXCXCXXA | 24 | 788-811 | E7 |
| HPV6b | GACGAAGXGGACGGACAAGAXXC | 23 | 641-663 | E7 |
| HPV8 | XGXCAACGCAACXGAXXCGGGXAX | 24 | 856-879 | E7 |
| HPV11 | XGAGGXGGACAAGGXGGACAAAC | 23 | 634-656 | E7 |
| HPV16 | AGXGXGACXCXACGCXXCGGXXG | 23 | 740-762 | E7 |
| HPV18 | XXGXAAGXGXGAAGCCAGAAXXGAG | 25 | 784-808 | E7 |
| HPV33 | AGCAAGXGACCXACGAACCAXACA | 24 | 788-811 | E7 |

Un autre objet de la présente invention est une nouvelle série de sondes d'acides nucléiques spécifiques de la région E7 de chacun des types de virus de HPV par rapport aux autres types de virus, ces sondes comportant des oligonucléotides de longueur de 35 monomères, à savoir les oligomères suivants ou leurs complémentaires :

| Virus | Oligomère | Position |
|---|---|---|
| HPV1a | GACCGXCCXCGCGGAXCACAGCGCCAXXAGACAGC | 711-745 |
| HPV5 | GAGGAGCCXGACAACGAAAGGAXCXCXXACAAAGX | 783-817 |
| HPV6b | GAGGXGGACGAAGXGGACGGACAAGAXXCACAACC | 635-669 |
| HPV8 | CXXXXXGXCAACGCAACXGAXXCGGGXAXCAGGAC | 851-885 |
| HPV11 | AGAAGAXGAGGXGGACAAGGXGGACAAACAAGACG | 628-662 |
| HPV16 | XXGCAAGXGXGACXCXACGCXXCGGXXGXGCGXAC | 735-769 |
| HPV18 | AXGXGXXGXAAGXGXGAAGCCAGAAXXGAGCXAGX | 779-813 |
| HPV33 | AGXACAGCAAGXGACCXACGAACCAXACAGCAACX | 783-817 |

Ces séries de sondes correspondent comme les précédentes à la fibre codante de l'ADN du virus et leurs complémentaires à la fibre inverse qui peuvent donc s'hybrider avec l'ARN messager notamment. Les positions indiquées correspondent au brin direct comme pour les précédentes.

La présente invention concerne également des sondes qui permettent de distinguer les virus HPV responsables des tumeurs de peau de ceux, propres aux muqueuses, qui provoquent des cancers anogénitaux ou des voies orales, nasales et paranasales.

La présente invention a donc pour objet des sondes spécifiques du sous-groupe de virus constitué par HPV16, HPV18 et HPV33 qui provoquent des tumeurs de muqueuses, caractérisées en ce qu'elles comportent la séquence suivante :

XXAGGCACAXAXXXX

ou son complémentaire.

Cette séquence est commune à HPV16, HPV18 et HPV33 et ne se retrouve pas dans les autres types de virus HPV.

Elle se situe aux positions suivantes (numérotation par rapport à la fibre codante)

| Virus | Position | Gène |
|---|---|---|
| HPV16 | 7698-7712 | en aval de L1 |
| HPV18 | 7686-7700 | en aval de L1 |
| HPV33 | 7741-7755 | en aval de L1 |

La présente invention a également pour objet des sondes spécifiques du sous-groupe HPV16, HPV18 qui provoquent des tumeurs de muqueuses, caractérisées en ce qu'elles comportent la séquence suivante :

AXAXCAAAXAXXAGXGAAGX

ou son complémentaire.

Cette séquence est commune à HPV16 et HPV18 et ne se retrouve dans aucun des autres types de virus HPV.

Elle se situe aux positions suivantes (numérotation par rapport à la fibre codant)

| Virus | Position | Gène |
|---|---|---|
| HPV16 | 1840-1859 | E1 |
| HPV18 | 1910-1929 | E1 |

Enfin, la présente invention a pour objet une sonde spécifique du sous-groupe de virus consistant en HPV5 et HPV8 qui provoque des tumeurs de la peau, caractérisée en ce qu'elle comporte un fragment de 56 bases de long dont la séquence définie par rapport au brin codant est la suivante :

Elle se situe aux position suivantes (numérotation par rapport à la fibre codande)

| Virus | Position | Gène |
|-------|----------|------|
| HPV5 | 4450-4505 | L2 |
| HPV8 | 4383-4438 | L2 |

A l'intérieur de ce fragment, on peut choisir des séquences plus courtes ayant des températures de demi-hybridation de l'ordre de 70°C. Parmi ces différentes possibilités, on peut citer la sonde comportant la séquence

XAXGGCAGXGCXGGXGXAXXXXXXG

ou son complémentaire

Cette séquence correspond aux positions 4480-4504 de HPV5 et 4413-4437 de HPV8 (numérotées par rapport à la fibre codante).

Les sondes sélectionnées, communes à chacun des sous-groupes de virus HPV, permettent d'identifier chacun des sous-groupes et, par leur sélectivité, de les distinguer entre eux.

La présente invention a également pour objet une sonde d'acides nucléiques, caractérisée en ce qu'elle est marquée par l'un des procédés connus pour le marquage des sondes ADN ou ARN, choisi notamment parmi les marquages radioactifs tels que $^{32}$P et les marquages enzymatiques, tels qu'avec la biotine, ainsi qu'un procédé de détection d'un type de virus HPV ou d'un sous-groupe de virus HPV à l'aide d'une sonde, selon l'invention, caractérisé en ce qu'on hybride ladite sonde à une séquence d'ADN ou d'ARN desdits virus HPV, le cas échéant, préalablement dénaturé s'il était initialement bicaténaire.

D'autres avantages et caractéristiques de la présente invention apparaîtront à la lumières de la description qui va suivre.

Sur les figures annexées,

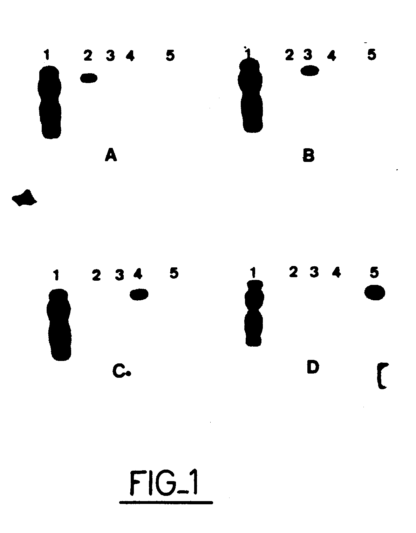

. la figure 1 représente un gel correspondant à l'hybridation sélective des sondes synthétiques HPV selon l'invention aux ADN des virus HPV :

1. fragments d'ADN radioactifs lambda EcoRI-HindIII - standards de taille,

2. ADN du virus HPV6b, digéré par BamHI,

3. ADN du virus HPV11, digéré par BamHI,

4. ADN du virus HPV16, digéré par BamHI,

5. ADN du virus HPV18, digéré par EcoRI,

A. sonde spécifique de HPV6b,

B. sonde spécifique de HPV11,

C. sonde spécifique de HPV16,

D. sonde spécifique de HPV18 ;

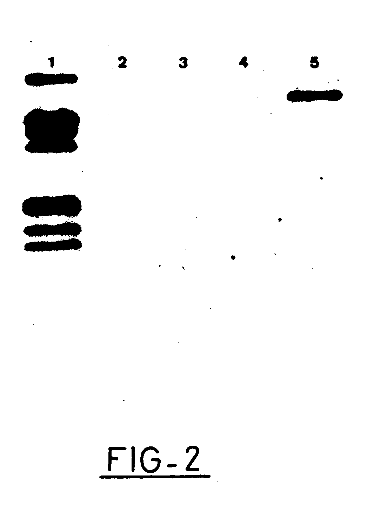

. la figure 2 représente un gel correspondant à l'hybridation de l'ADN génomique avec des sondes HPV spécifiques selon l'invention :

1. lambda EcoRI-HindIII - standards de taille radioactifs,

2. rien,

3. ADN génomique humain (10 $\mu$g) digéré par EcoRI,

4. rien,

5. ADN du virus HPV (6b, 11, 16 ou 18) hybridé avec sonde spécifique HPV (6b, 11, 16 ou 18) ;

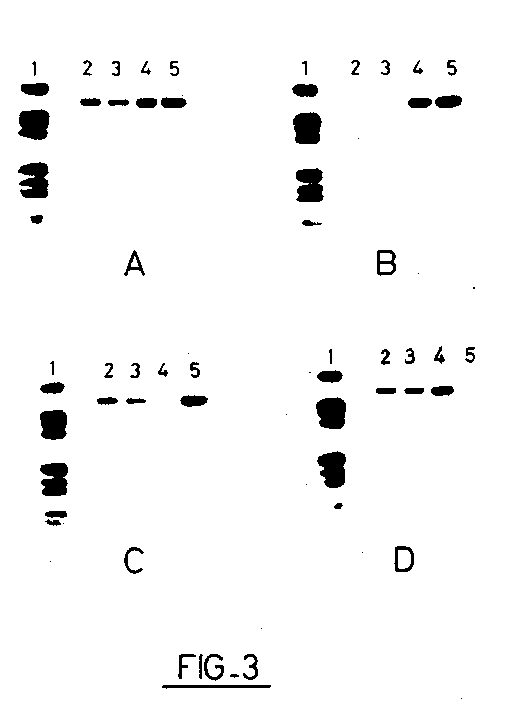

. la figure 3 représente un gel correspondant à l'hybridation des clones HPV aux sondes communes à tous les types de virus.

La figure 4 représente l'hybridation sélective des sondes spécifiques synthétiques HPV aux ADN des virus HPV.

a : ADN du virus HPV6b, digéré par BamHI

b : ADN du virus HPV11, digéré par BamHI

c : ADN du virus HPV16, digéré par BamHI

d : ADN du virus HPV18, digéré par EcoRI

A : sonde spécifique de HPV6b

B : sonde spécifique de HPV11

C : sonde spécifique de HPV16

D : sonde spécifique de HPV18

La figure 5 représente l'hybridation sélective des sondes synthétiques dirigées contre la région E7 du génome de HPV aux ADN des virus HPV.

a : ADN du virus HPV6b, digéré par BamHI

b : ADN du virus HPV11, digéré par BamHI
c : ADN du virus HPV16, digéré par BamHI
d: ADN du virus HPV18, digéré par EcoRI
A : sonde spécifique de HPV6b
B : sonde spécifique de HPV11
C : sonde spécifique de HPV16
D : sonde spécifique de HPV18
1 : fragments d'ADN radioactifs lambda EcoRI-HindIII-Standards de taille.

EXEMPLE 1

Détermination de sondes oligonucléotidiques communes à tous les virus de papillomes humains connus

En vue de la préparation d'une sonde permettant le diagnostic rapide des affections à base de virus de papillome, il a été procédé à la recherche des séquences les plus longues qui soient communes à tous les virus dont la séquence est aujourd'hui connue, à savoir à ce jour des séquences des virus suivants : HPV1a, HPV5, HPV6b, HPV8, HPV11, HPV16, HPV18 et HPV33.

Un programme FORTRAN a été développé spécifiquement en vue du travail considéré, les algorithmes usuels étant impropres à la manipulation de plusieurs séquences aussi étendues. Le programme explore chacune des séquences qui lui sont soumises au moyen de tous les blocs possibles extraits de la première d'entre elles, ces blocs prenant successivement toutes les valeurs entre 20 et 10 bases de long. Au cours de cette recherche, le programme annonce les occurrences des blocs intégralement conservés au travers de l'ensemble des séquences qui lui sont soumises.

La plus longue séquence commune trouvée, à savoir le fragment de douze bases (T-A-A-A-A-C-G-A-A-A-G-T) est située aux positions suivantes dans les diverses séquences :

| | |
|---|---|
| HPV1a | 1029 |
| HPV5 | 1190 |
| HPV6b | 1076 |
| HPV8 | 1174 |
| HPV11 | 1076 |
| HPV16 | 1121 |
| HPV18 | 1167 |
| HPV33 | 1132 |

Comme douze bases constituent un fragment trop court pour pouvoir servir aisément dans certaines conditions expérimentales, on a rallongé cette séquence vers l'amont comme vers l'aval.

Un prolongement de quatre bases de chaque côté ne fournit que des séquences donnant des températures de demi-hybridation de l'ordre de 50-52°C. On a donc prolongé de six ou sept bases tant vers l'amont que vers l'aval, chaque fois par les séquences exactes présentes dans les virus pour donner ce qui suit :

| Séquence cible | Position | Température |
|---|---|---|
| HPV1a : ATGCTTTAAAACGAAAGTTACTTT | 1023 | 60°C |
| HPV5 : CAAAAACTAAAACGAAAGTATCTTA | 1183 | 62°C |
| HPV6b : AGGACCTAAAACGAAAGTATTTAG | 1070 | 64°C |
| HPV8 : CAAAAACTAAAACGAAAGTATCTTA | 1167 | 62°C |
| HPV11 : AGGACCTAAAACGAAAGTATTTAG | 1070 | 64°C |
| HPV16 : AGGTTCTAAAACGAAAGTATTTGG | 1115 | 64°C |
| HPV18 : ATGTTTTAAAACGAAAGTTTGCAG | 1161 | 62°C |
| HPV33 : GTGCACTAAAACGAAAGTTTGCCG | 1126 | 70°C |

Certaines de ces sondes peuvent être quelque peu raccourcies et celle spécifique du HPV8 peut encore éventuellement être rallongée pour obtenir des températures de demi-dénaturation quasi identiques.

Il est à noter que les séquences obtenues pour HPV6b et HPV11 sont identiques, de même que pour HPV5 et HPV8. Le mélange d'oligonucléotiques ne comporte donc que six molécules différentes.

Le mélange de ces six oligonucléotides permet d'obtenir, pout tout système comportant de l'ADN d'un des virus de papillomes humains, un signal d'hybridation bien caractéristique.

EXEMPLE 2

Détermination de sondes spécifiques de chacun des types de HPV connus (première série de sondes)

Le travail a consisté en la recherche de sondes oligonucléotidiques de longueur avoisinant vingt bases, et choisies de telle sorte qu'elles soient chacune spécifiques de l'un ou l'autre des huit virus de papillomes humains dont les séquences complètes sont aujourd'hui connues. Leur finalité est d'être utilisées comme éléments sélectifs pour des tests diagnostics d'infections par ces virus.

A cette fin a été développé un logiciel spécifique, comportant des programmes FORTRAN, et destiné à la comparaison de plusieurs séquences de grande taille, en vue de la détection des régions les moins conservées. Ce programme fonctionne comme un filtre progressif, marquant à chacune de ses exécutions successives les régions d'une séquence qui se retrouvent exactement dans chacune des autres séquences considérées. Après chaque passage, la longueur de l'homologie recherchée est décrémentée d'une unité, entre la valeur initiale de 25 et une valeur finale de 8.

Chacune des versions de la séquence de référence, marquée lors de chacun des passages successifs avec chacune des séquences cibles, est stockée séparément. Un second programme détermine alors entre les diverses séquences marquées, les régions non marquées, et ceci pour chacun des stades successifs du filtrage. A la sortie, on obtient une liste des régions potentiellement utilisables. Le choix parmi ces régions s'opère par un examen de l'environnement de la région lors des passages précédents du filtre, de telle sorte que l'oligomère choisi présente un minimum d'homologies avec les séquences d'ADN des autres virus .

On est arrivé ainsi, par exemple, à la première série d'oligomères suivants, chacun dénommé en fonction du virus pour lequel il est sélectif :

| Virus | Oligomère | Longueur (bases) | Position | Température |
|---|---|---|---|---|
| HPV1a | CCTCAGGCGAGAAGCGCGGAC | 21 | 1052 | 72°C |
| HPV5 | TGTATGGGATCGGGGGCGTTTA | 22 | 1801 | 68°C |
| HPV6b | CATTAACGCAGGGGCGCCTGAA | 22 | 4761 | 70°C |
| HPV8 | ACACCGCCGCCAAGACCCCCA | 21 | 3344 | 72°C |
| HPV11 | GGCGTGTCGGCGCCGCCTAG | 20 | 3416 | 72°C |
| HPV16 | CAGAACCGGACAGAGCCCAT | 20 | 695 | 64°C |
| HPV18 | CGGTATCCGCTACTCAGCTTG | 21 | 3448 | 64°C |
| HPV33 | CGTCCTCCGGTTACTGTAGACA | 22 | 4474 | 68°C |

Chacune des sondes proposées ci-dessus a ensuite été comparée à chacune des séquences HPV par l'algorithme de Sellers et Goad, qui cote les "mismatches"[1] et les "gaps"[2]. Les résultats de ce traitement montrent que les sondes choisies n'ont jamais plus de 75 % d'homologies avec les séquences non

[1] "mismatch": appariement de bases impropre à la formation de liens hydrogènes ; cet appariement ne contribue donc pas à la stabilisation de la région hybridée et aura par contre fréquemment un effet déstabilisant.

[2] "gap": lors de la tentative d'appariement de deux régions d'acides nucléiques, il peut arriver qu'afin de maximiser la stabilité globale de la région hybridée, il soit nécessaire de prévoir dans une des séquences l'existence d'une ou plusieurs bases non appariées ; ce genre de "saut" détermine généralement une contribution négative à la stabilité globale de l'hybride.

homologues. Les résultats obtenus sont présentés dans le tableau I ci-après.

Dans chaque case sont repris :
- le nombre de bases appariables
- le nombre de"mismatches"
- Le nombre de"gaps"

TABLEAU I

| Séquence cible de : | HPV1a | HPV5 | HPV6b | HPV8 | HPV11 | HPV16 | HPV18 | HPV33 |
|---|---|---|---|---|---|---|---|---|
| Sonde de : | | | | | | | | |
| HPV1a | 21/0/0 | -de 8 | 15/1/2 | 10/0/1 | 13/1/2 | 9/0/1 | -de 8 | 13/1/2 |
| HPV5 | -de 8(*) | 22/0/0 | -de 8 | 14/1/1 | -de 8 | 12/3/0 | -de 8 | 10/0/1 |
| HPV6b | 11/2/0 | 15/2/1 | 22/0/0 | 12/3/0 | 16/4/0 | 10/0/1 | -de 8 | -de 8 |
| HPV8 | 9/0/1 | 17/4/0 | 9/1/0 | 21/0/0 | 12/1/1 | 9/1/0 | -de 8 | 8/0/0 |
| HPV11 | -de 8 | 11/2/0 | 12/1/0 | 11/1/0 | 22/0/0 | 11/2/0 | 10/1/0 | 10/1/0 |
| HPV16 | 13/1/2 | 12/2/0 | 15/3/1 | 11/2/0 | -de 8 | 20/0/0 | 12/1/1 | - de 8 |
| HPV18 | 10/0/1 | 13/1/2 | 12/2/0 | 10/0/1 | 13/3/0 | 13/3/0 | 21/0/0 | 10/1/0 |
| HPV33 | 10/1/0 | 11/0/2 | 11/2/0 | 12/3/0 | - de 8 | -de 8 | 11/2/0 | 22/0/0 |

(*) moins de 8 bases appariables.

Toutes les situations garantissent des hybridations hautement spécifiques, sans risque de signaux erronés ou de bruits de fond gênants.

EXEMPLE 3

Hybridation des sondes HPV avec l'ADN des différents virus

Les génomes de l'HPV6b, 11, 16 et 18 ont été reçus clonés dans pBR322. Quand on digère les clones 6b, 11, 16 par BamHI et le clone 18 par EcoRI, on sépare le génome du virus (environ 8 kb) du pBR322.

a) Amplification des clones

La présence des génomes d'HPV dans les clones a été confirmée par restriction. Les clones ont ensuite été amplifiés et l'ADN a été purifié sur gradient de chlorure de césium.

b) Séparation de l'ADN des virus HPV sur gel d'agarose

2 $\mu$g de chaque ADN ont été digérés soit par BamHI pour les clones 6b, 11 et 16, soit par EcoRI pour le clone 18. Les 2 $\mu$g de chaque ADN ont été répartis en adjacence et parallèlement sur quatre gels d'agarose 0,8 % (500 ng ADN/clone/gel) à proximité d'un marqueur de taille marqué radioactivement (lambda HindIII-EcoRI). Après 3 heures de migration à 80 Volts dans du 1XTBE (Tris 90 mM, EDTA 2,5 mM, acide borique 90 mM, pH 8), les gels ont été dénaturés (NaOH 0,5 M, NaCl 0,5 M) pendant 30 minutes, rincés à l'eau puis neutralisés (NaCl, 1,5 M, Tris HCl pH 8, 0,5 M) pendant 30 minutes à température ambiante. Les gels ont ensuite été déposés sur du papier Whatman 3 MM, recouverts de saranwrap et mis à sécher sous vide pendant 1 heure à température ambiante et 1 heure à 60°C.

c) Marquage au $^{32}$P des sondes HPV spécifiques

Des oligomères synthétiques caractéristiques des HPV6b, 11, 16 et 18 ont été synthétisés.

|                                              | Taille | Température |
|----------------------------------------------|--------|------------|
| HPV6b : CATTAACGCAGGGGCGCCTGAA               | 22 mer | 70°C       |
| HPV11 : GGCGTGTCGGCGCCGCCTAG                 | 20 mer | 72°C       |
| HPV16 : CAGAACCGGACAGAGCCCAT                 | 20 mer | 64°C       |
| HPV18 : CGGTATCCGCTACTCAGCTT                 | 20 mer | 60°C       |

Ces séquences sont complémentaires à la fibre non codante des HPV.

Les quatre oligomères ont été marqués au $^{32}$P par kination selon le protocole suivant : 1 $\mu$g de chaque oligomère a été mélangé, dans un volume finale de 30 $\mu$l, à 30 $\mu$Ci de gamma $^{32}$P ATP (3 000 Ci/mmole), 5 unités T4 polynucléotide kinase, 67 mM Tris HCl pH 8, 10 mM MgCl$_2$, 10 mM dithiothréitol et incubé 45 minutes à 37°C. Les oligomères marqués ont été séparés des nucléotides libres par chromatographie sur Sephadex® G50. L'activité spécifique obtenue s'élève à 65.10$^6$ cpm/$\mu$g d'oligomère engagé dans la réaction.

d) Hybridation sur gel avec les sondes marquées

Les gels d'agarose ont été décollés du papier Whatman® en les plongeant quelques minutes dans de l'eau. Chaque gel a été hybridé avec l'un des quatre oligomères marqués radioactivement, dans un volume de 5 ml de mélange d'hybridation comprenant 2.10$^6$ cpm/ml d'oligomères radioactifs, 0,1 % SDS, 100 $\mu$g/ml d'ADN de sperme de saumon dénaturé 10 minutes à 100°C, 5 x SSPE (NaCl 0,9 M, NaH$_2$PO$_4$, EDTA pH 8, 5 mM). L'incubation s'effectue dans des sacs en plastique scellés et dans un bain agitant à 60°C pendant 14 heures.

e) Lavage et exposition

Les gels ont été lavés deux fois 15 minutes, puis 1 heure à température ambiante, 10 minutes à 60°C et enfin 1 heure à température ambiante dans du 6xSSC (0,9 M NaCl, 90 mM citrate trisodique, pH 7). Après avoir été déposés sur une feuille de papier Whatman® et recouverts de saranwrap, les gels ont été mis à exposer entre deux écrans renforçateurs à -70°C pendant 14 heures (Film Kodak® X-OmatS).

f) Résultats

Quatre duplicata d'un gel contenant l'ADN des clones HPV6b, 11, 16 et 18 ont été chacun hybridés à l'un des quatre oligomères. On voit que sur le gel A seul l'HPV6b est reconnu par la sonde 6b ; sur le gel B, seul l'HPV11 est reconnu par la sonde 11; sur le gel C, seul l'HPV16 est reconnu par la sonde 16 et enfin sur le gel D, seul l'HPV18 est reconnu par la sonde 18.

Il en résulte que chaque sonde reconnaît exclusivement l'HPV correspondant à l'exclusion des autres types d'HPV (figure 1).

Hybridation des sondes HPV sur l'ADN génomique humain

Par ailleurs, on a déposé sur 4 gels, 4 x 10 $\mu$g d'ADN génomique isolé de lymphocytes, digérés par EcoRI, et 200 ng d'ADN d'un des 4 clones d'HPV. Chaque gel a été hybridé à la sonde correspondant au type d'HPV présent sur le gel (témoin positif) et lavé comme décrit précédemment. Après 70 heures d'exposition, on ne décèle absolument aucune trace d'hybridation aspécifique des quatre types de sondes testées sur l'ADN génomique humain (figure 2).

EXEMPLE 4

Hybridation des clones HPV aux sondes communes à tous les types d'HPV connus

a) Préparation des gels

Les clones des HPV 6b, 11, 16 et 18 ont été digérés par BamHI ou EcoRI. 600 ng de chaque clone ont été répartis parallèlement sur quatre gels et en adjacence aux autres clones, et à proximité du marqueur de taille radioactif, le lambda HindIII-EcoRI (150 ng ADN/clone/ gel). Après 3 heures de migration à 80 Volts, les gels ont été dénaturés, neutralisés et séchés sous vide.

b) Kination des sondes communes (marquage au $^{32}$P)

On a déterminé, par voie informatique, la séquence la plus longue qui est commune à tous les types de virus dont la séquence est connue. Cette séquence commune est un fragment de 12 bases. Ce fragment étant trop court, les sondes ont été prolongées de 6 bases (7 bases pour la sonde 8) en amont, et de 6 bases en aval de ce fragment. Les séquences sont les suivantes :

| | Sonde | Taille | Température |
|---|---|---|---|
| HPV1a | ATGCTTTAAAACGAAAGTTACTTT | 24 | 60°C |
| HPV6b HPV11 | AGGACCTAAAACGAAAGTATTTAG | 24 | 64°C |
| HPV8 HPV5 | CAAAAACTAAAACGAAAGTATCTTA | 25 | 62°C |
| HPV16 | AGGTTCTAAAACGAAAGTATTTGG | 24 | 64°C |
| HPV18 | ATGTTTTAAAACGAAAGTTTGCAG | 24 | 62°C |
| HPV33 | GTGCACTAAAACGAAAGTTTGCCG | 24 | 70°C |

Ces sondes sont complémentaires à la fibre codante. Comme les sondes 6b et 11 sont parfaitement identiques, seule une des deux a été synthétisées (sonde 6b/11). Ces sondes ont été marquées au $^{32}$P par kination comme décrit précédemment.

c) Hybridation des gels

Chaque gel a été hybridé avec un lot de sonde, dans un volume de 5 ml de mélange d'hybridation contenant 0,1 % SDS, 100 $\mu$g/ml ADN de sperme de saumon dénaturé, 5xSSPE dans un sac en plastique scellé, à 60°C pendant 15 heures.

Le mélange d'hybridation A contenait $10^6$ cpm/ml de chacune des sondes (1a, 6b/11, 8/5, 16, 18 et 33).
Le mélange B contenait $10^6$ cpm/ml de chacune des sondes à l'exception de la sonde 6b/11.
Le mélange C contenait $10^6$ cpm:ml de chacune des sondes à l'exception de la sonde 16.
Le mélange D contenait $10^6$ cpm/ml de chacune des sondes à l'exception de la sonde 18.

d) Lavage et exposition

Les quatre gels ont été lavés dans du 6XSSC une heure à température ambiante, 10 minutes à 60°C, puis de nouveau une heure à température ambiante. Les gels ont été exposés entre deux écrans renforçateurs pendant deux heures à -80°C.

e) Résultats (figure 3)

Hybridation des clones HPV aux sondes communes à tous les types de virus.
1. lambda HindIII-EcoRI,
2. HPV6b BamHI,
3. HPV11 BamHI,
4. HPV16 BamHI,
5. HPV18 EcoRI
A. Hybridation à l'ensemble des sondes (1a, 6b/11, 16, 18, 33),
B. Hybridation à l'ensemble des sondes à l'exception de la sonde 6b/11,
C. Hybridation à l'ensemble des sondes à l'exception de la sonde 16,
D. Hybridation à l'ensemble des sondes à l'exception de la sonde 18.
Ces résultats montrent clairement que les sondes communes s'hybrident avec l'ensemble des clones en expérience et que les conditions d'expérimentation permettent même de différencier les clones par la spécificité des extrémités d'extension de la séquence commune.

EXEMPLE 5

Hybridation de sondes spécifiques HPV avec l'ADN des différents virus (Seconde et troisième séries de sondes)

Les génomes de l'HPV6b, 11, 16 et 128 ont été reçus clonés dans pBR322. Quand on digère les clones 6b, 11, 16 par BamHI et le clone 18 par EcoRI, on sépare le génone du virus (environ 8 kb) du pBR322.

a)Amplification des clones

La présence des génomes d'HPV dans les clones a été confirmée par restriction. Les clones ont ensuite été amplifiés et l'ADN a été purifié sur gradient de chlorure de césium.

b) Séparation de l'ADN des virus HPV sur gel d'agarose

1,6 μg de chaque ADN ont été digérés soit par BamHI pour les clones 6b, 11 et 16, soit par EcoRI pour le clone 18. Les 1,6 μg de chaque ADN ont été répartis en adjacence et parallèlement sur huit gels d'agarose 0,8 % (200 ng ADN/clone/gel) à proximité d'un marqueur de taille marqué radioactivement (lambda HindIII-EcoRI). Après 3 heures de migration à 80 Volts dans du 1XTBE (Tris 90 mM, EDTA 2,5 mM, acide borique 90 mM, pH 8), les gels ont été dénaturés (NaOH 0,5 M, NaCl 1,5 M) pendant 30 minutes, rincés à l'eau, puis neutralisés (NaCl 1,5 M, Tris HCl pH 8, 0,5 M) pendant 30 minutes à température ambiante. Les gels ont ensuite été déposés sur du papier Whatman® 3MM, recouverts de saranwrap et mis à sécher sous vide pendant 1 heure à température ambiante et 1 heure à 60°C.

c) Marquage au $^{32}$P des sondes HPV spécifiques

Les oligomères synthétiques caractéristiques des HPV6b, 11, 16 et 18 ont été synthétisés.
Il s'agit de la seconde série de sondes spécifiques :

| Virus | Oligomère | Longueur | Position | Gène | Temp. |
|-------|-----------|----------|----------|------|-------|
| HPV6b | CGCCCCTGCGTTAATGATTGCC | 22 | 4776-4755 | L2 | 70°C |
| HPV11 | AATAGCAGGCTTGGGAGAGCTTC | 23 | 4659-4637 | L2 | 70°C |
| HPV16 | CAGTAGAGATCAGTTGTCTCTGGT | 24 | 632-609 | E7 | 70°C |
| HPV18 | GGGCCGTGGGTGATACAATGGG | 22 | 5507-5486 | L2 | 72°C |

Par ailleurs, une troisième série de sondes chacune spécifique d'un type de virus du papillome humain, mais située dans la région E7 du génome de chacun de ces virus, a été parallèlement synthétisée.
Série sondes E7 :

14

| Virus | Oligomère | Longueur | Position | Gène | Temp. |
|-------|-----------|----------|----------|------|-------|
| HPV6b | GAATCTTGTCCGTCCACTTCGTC | 23 | 663-641 | E7 | 70°C |
| HPV11 | GTTTGTCCACCTTGTCCACCTCA | 23 | 656-634 | E7 | 70°C |
| HPV16 | CAACCGAAGCGTAGAGTCACACT | 23 | 762-740 | E7 | 70°C |
| HPV18 | CTCAATTCTGGCTTCACACTTACAA | 25 | 808-784 | E7 | 70°C |

Dans ces deux séries, les sondes correspondent à la fibre inverse des HPV, les positions indiquées sont repérées sur la fibre directe.

Les huit oligomères ont été marqués au $^{32}$P par kination selon le protocole suivant : 0,1 $\mu$g de chaque oligomère a été mélangé, dans un volume final de 30 $\mu$l, à 30 $\mu$Ci de gamma $^{32}$P ATP (3000 Ci/mmole), 5 unités T4 polynucléotide kinase, 67 mM Tris HCl pH 8, 10 mM MgCl$_2$, 10 mM dithiothrétiol et incubé 45 minutes à 37°C. Les oligomères marqués ont été séparés des nucléotides libres par chromatographie sur Sephadex® G50. L'activité spécifique obtenue s'élève à 5.10$^8$ cpm/$\mu$g d'oligomère engagé dans la réaction.

d) Hybridation sur gel avec les sondes marquées

Les gels d'agarose ont été décollés du papier Whatman® en les plongeant quelques minutes dans de l'eau. Chaque gel a été hybridé avec l'un des quatre oligomères marqués radioactivement, dans un volume de 5 ml de mélange d'hybridation comprenant 2.10$^6$ cpm/ml d'oligomères radioactifs, 0,1 % SDS, 100 $\mu$g/m d'ADN de sperme de saumon dénaturé 10 minutes à 100°C, 5 x SSPE (NaCl 0,9 M, NaH$_2$PO$_4$ 50 mM, EDTA pH 8, 5 mM). L'incubation s'effectue dans des sacs en plastique scellés et dans un bain agitant à 65°C pendant environ 14 heures.

e) Lavage et exposition

Les gels ont été lavés deux fois 15 minutes, puis 1 heure à température ambiante, 10 minutes à 65°C et enfin 1 heure à température ambiante dans du 6XSSC (0,9 M NaCl, 90 mM citrate trisodique, pH 7). Après avoir été déposés sur une feuille de papier Whatman® et recouverts de saranwrap, les gels ont été mis à exposer entre deux écrans renforçateurs à -70°C de 2 à 14 heures (Film Kodak® X-OmatS).

f) Résultats

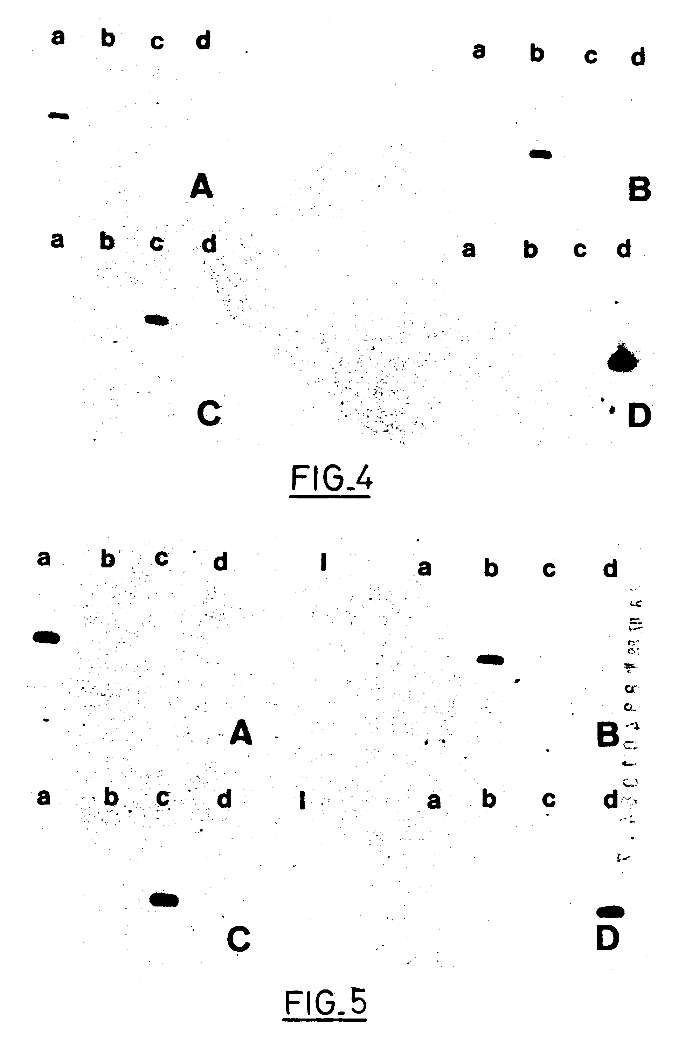

Deux séries de quatre duplicata contenant l'ADN des clones HPV6b, 11, 16 et 18 ont été chacune hybridée à l'un des quatre oligomères des deux séries sondes spécifiques. On voit que l'HPV6b n'est reconnu que par les sondes 6b (Fig. 4A et 5A), que l'HPV11 n'est reconnu que par les sondes 11 (Fig. 4b et 5B), que l'HPV16 n'est reconnu que par les sondes 16 (Fig. 4C et 5C) et enfin que seul l'HPV18 est reconnu par les sondes 18 (Fig. 4D et 5D). Chaque sonde reconnaît exclusivement l'HPV correspondant à l'exclusion des autres types de HPV.

EXEMPLE 6 :

En vue d'augmenter la sensiblité de la détection des infections par les HPVs, la technique d'amplification enzymatique de l'ADN couramment dénommée PCR (polychain reaction) dans la littérature scientifique de la spécialité a été appliquée à l'amplification des ADN des HPVs.

Cette démarche vise à l'augmentation de la sensibilité tout en conservant la spécificité de la détection illustrée plus haut dans ce brevet. La région des génomes viraux choisie pour l'amplification comporte par conséquent les séquences de base complémentaires aux sondes destinées à la détection du gène E7 des divers virus. La spécificité de l'amplification est assurée par la définition de séquences amorces de la polymérisation par une polymérase thermostable de part et d'autre de la séquence cible des sondes. Les séquences de chacune des paires d'amorces sont donc choisies de manière à amplifier spécifiquement le fragment du gène du virus auquel elles s'adressent, à l'exclusion de tous les autres. En outre, la position relative de chacune des amorces par rapport à sa paire a été choisie de manière à amplifier des fragments de taille différente pour chaque ADN viral et à permettre l'identification rapide et directe du type ou des types de virus de papilloma humain par séparation des fragments amplifiés en fonction de leurs longueurs, sur gel d'électrophorèse. Un critère supplémentaire du choix des séquences des amorces tient compte de leur composition et de leur longueur qui doivent permettre une hybridation spécifique avec la séquence complémentaire de l'ADN à amplifier, à des températures de fonctionnement optimales des polymérases thermostables.

Les séquences suivantes, destinées à servir d'amorces oligomériques pour les réactions d'amplification enzymatique ont été définies compte tenu des critères mentionnés ci-dessus.

| | position | longueur du fragment amplifié |
|---|---|---|
| **pour HPV1a** | | |
| GTTGCTTCCTGTGCCTATTGCG | 673-694 | |
| | | 90 |
| CAGAAGGAGTTCCTCCAGCTG | 762-742 | |
| **pour HPV5** | | |
| ATTATTCTGGAGCTCAGTGAGGT | 693-715 | |
| | | 252 |
| ACAGTCAGGGCACAGGAGCTGC | 944-922 | |
| **pour HPV6B** | | |
| TGTATTAGACCTGCAACCTCCAG | 562-584 | |
| | | 224 |
| TTCCCAACAGAAGCTGTTGCAC | 785-764 | |

pour HPV8

| | | |
|---|---|---|
| CTAGACATCGAAAGAACTGTATTC | 779-802 | |
| GCACTCAGGACACAGAAGCTGT | 934-913 | 156 |

pour HPV11

| | | |
|---|---|---|
| AGTACTAGACCTGCAGCCTCCT | 562-583 | |
| GTAGTTGTCTGATGTCTCCGTC | 767-746 | 206 |

pour HPV16

| | | |
|---|---|---|
| GCAGAACCGGACAGAGCCCA | 694-713 | |
| GTGTGCCCATTAACAGGTCTTCC | 820-798 | 127 |

pour HPV18

| | | |
|---|---|---|
| GCCCGACGAGCCGAACCACA | 740-759 | |
| GGAATGCTCGAAGGTCGTCTG | 848-828 | 109 |

pour HPV33

| | | |
|---|---|---|
| GGCTTGGACCGGCCAGATGG | 681-700 | |
| GTGCACAGGTAGGGCACACAA | 858-838 | 178 |

Séquence du virus HPV1A:
Danos, O., Katinka, M., Yaniv, M. (1982) EMBO J. 1, p.231-236
Base de données EMBL: V01116

Séquence du virus HPV5:
Zachow, K., Ostrow, R.S., Faras, A.J. (1987) Virology, 158, p. 251-254
Base de données EMBL: M22961

Séquence du virus HPV6B:
Schwartz, E., Dürst, M., Demankowski, C., Latterman, O., Zech, R., Wolfsperger, E., Suhai, S., zur Hausen, H. (1983) Nature 314 p.2341-2348
Base de données EMBL: X00203

Séquence du virus HPV8:
Fuchs, P.G., Iftner, T., Weninger, J., Pfister, H. (1986) J. Virol. 58, p.626-634
Base de données EMBL: M12737

Séquence du virus HPV11:
Dartman, K., Schwartz, E., Gissman, L., zur Hausen, H. (1986) Virology 151, p.124-130

Base de données EMBL: M14119

Séquence du virus HPV16:
Seedorf, K., Krämmer, G., Dürst, M., Suhai, S., Röwekamp, W.G. (1985)
Virology, 145, p. 181-185
Base de données EMBL: K02718

Séquence du virus HPV18:
Cole, S.T., Danos, O. (1987) J.Mol. Biol. 193, p.599-608
Base de données EMBL: X05015

Séquence du virus HPV33:
Cole, S.T., Streeck, R.E. (1986) J. Virol. 58, p. 991-995
Base de données EMBL: M12732

**Revendications**

1. Sonde d'acide nucléique utile pour détecter indifféremment les différents types de virus de papillomes humains, notamment HPV1a, HPV5, HPV6b, HPV8, HPV11, HPV16, HPV18 et HPV33 consistant en une séquence d'acides nucléiques marquée, caractérisée en ce qu'elle comprend l'oligomère de douze nucléotides X-A-A-A-C-G-A-A-A-G-X, avec X = T ou U, ou son complémentaire.

2. Mélange de six sondes d'acides nucléiques communes aux différents types de virus HPV consistant dans le mélange de six oligomères, lesquels comportent tous la séquence commune de douze nucléotides de la revendication 1, prolongée par les nucléotides correspondants pour chaque type de virus.

3. Mélange de six sondes d'acides nucléiques selon la revendication 2, caractérisée en ce que la séquence commune de douze nucléotides est prolongée de part et d'autre par 1 à 10 nucléotide(s) correspondant(s) pour chacun des huit types de virus.

4. Mélange de sondes selon la revendication 3, caractérisée en ce qu'il comprend le mélange des six oligomères suivants ou leurs complémentaires :

    pour HPV1a   A-X-G-C-X-X-X-A-A-A-A-C-G-A-A-AG-X-X-A-C-X-X-X

    pour HPV6b   A-G-GA-C-C-X-A-A-A-A-C-G-A-A-A-G-X-A-X-X-X-A-G
       et HPV11

    pour HPV8 C-A-A-A-A-A-C-X-A-A-A-A-C-G-A-A-A-G-X-A-X-C-X-X-A
       et HPV5

    pour HPV16   A-G-G-X-X-C-X-A-A-A-A-C-G-A-A-A-G-X-A-X-X-X-G-G

    pour HPV18   A-X-G-X-X-X-X-A-A-A-A-C-G-A-A-A-G-X-X-X-G-C-A-G

    pour HPV33   G-X-G-C-A-C-X-A-A-A-A-C-G-A-A-A-G-X-X-X-G-C-C-G

    avec X = T ou U.

5. Sonde d'acides nucléiques spécifique pour la détection du type de virus de papillome humain HPV1a, caractérisée en ce qu'elle comporte un oligomère choisi parmi :

CCXCAGGCGAGAAGCGCGGAC

CXGXCCCXXCXGCGCGAGACGX

XGXCCCXXCXGCGCGAGACGXXGGAAGXAXACACA

GACCGXCCXCGCGGAXCACAG

GACCGXCCXCGCGGAXCACAGCGCCAXXAGACAGC

avec X = T ou U
et leurs complémentaires.

6. Sonde d'acides nucléiques spécifique pour la détection du type de virus de papillome humain HPV5, caractérisée en ce qu'elle comporte un oligomère choisi parmi :

XGXAXGGGAXCGGGGGCGXXXA

**XX**GXAXGGGAXCGGGGGCGXXXA

XGXAXGGGAXCGGGGGCGXXXAGCCAXGGACCAXA

GCCXGACAACGAAAGGAXCXCXXA

GAGGAGCCXGACAACGAAAGGAXCXCXXACAAAGX

avec X = T ou U
et leurs complémentaires.

7. Sonde d'acides nucléiques spécifique pour la détection du type de virus de papillome humain HPV6b, caractérisée en ce qu'elle comporte un oligomère choisi parmi :

CAXXAACGCAGGGGCGCCXGAA

GGCAAXCAXXAACGCAGGGGCG

GCAAXCAXXAACGCAGGGGCGCCXGAAAXXGXGCC

GACGAAGXGGACGGACAAGAXXC

GAGGXGGACGAAGXGGACGGACAAGAXXCACAACC

avec X = T ou U
et leurs complémentaires.

8. Sonde d'acides nucléiques spécifique pour la détection du type de virus de papillome humain HPV8, caractérisée en ce qu'elle comporte un oligomère choisi parmi :

ACACCGCCGCCAAGACCCCCA

GCAGCXCAGXCCGCGGCXCC

GCAGCXCAGXCCGCGGCXCCAGXCAAXAXCACXGX

XGXCAACGCAACXGAXXCGGGXAX

CXXXXXGXCAACGCAACXGAXXCGGGXAXCAGGAC

avec X = T ou U

et leurs complémentaires.

9. Sonde d'acides nucléiques spécifique pour la détection du type de virus de papillome humain HPV11, caractérisé en ce qu'elle comporte un oligomère choisi parmi :

GGCGXGXCGGCGCCGCCXAG

GAAGCXCXCCCAAGCCXGCXAXX

XAXAXACCCXXGGGAAGCXCXCCCAAGCCXGCXAX

XGAGGXGGACAAGGXGGACAAAC

AGAAGAXGAGGXGGACAAGGXGGACAAACAAGACG

avec X = T ou U
et leurs complémentaires.

10. Sonde d'acides nucléiques spécifique pour la détection du type de virus de papillome humain HPV16, caractérisée en ce qu'elle comporte un oligomère choisi parmi :

CAGAACCGGACAGAGCCCAX

ACCAGAGACAACXGAXCXCXACXGX

et : ACAAGCAGAACCGGACAGAGCCCA

XGXXAGAXXXGCAACCAGAGACAACXGAXCXCXAC

AGXGXGACXCXACGCXXCGGXXG

XXGCAAGXGXGACXCXACGCXXCGGXXGXGCGXAC

avec X = T ou U
et leurs complémentaires.

11. Sonde d'acides nucléiques spécifique pour la détection du type de virus de papillome humain HPV18, caractérisée en ce qu'elle comporte un oligomère choisi parmi :

CCGXAXCCGCXACXCAGCXXG

CCCAXXGXAXCACCCACGGCCC

CCCAXXGXAXCACCCACGGCCCCXGCCXCXACACA

XXGXAAGXGXGAAGCCAGAAXXGAG

AXGXGXXGXAAGXGXGAAGCCAGAAXXGAGCXAGX

avec X = T ou U
et leurs complémentaires.

12. Sonde d'acides nucléiques spécifique pour la détection du type de virus de papillome humain HPV33, caractérisée en ce qu'elle comporte un oligomère choisi parmi :

CGXCCXCCGGXXACXGXAGAXA

GXCCXCCGGXXACXGXAGACACX

XCCXCCGGXXACXGXAGACACXGXXGGACCXXXAG

AGCAAGXGACCXACGAACCAXACA

AGXACAGCAAGXGACCXACGAACCAXACAGCAACX

avec X = T ou U
et leurs complémentaires.

**13.** Sonde d'acides nucléiques spécifique du sous-groupe de virus HPV constitué par HPV16, HPV18 et HPV33, caractérisé en ce qu'elle comporte l'oligomère

XXAGGCACAXAXXXX

avec X = T ou U
ou son complémentaire.

**14.** Sonde d'acides nucléiques spécifique du sous-groupe de virus constitué par HPV16 et HPV18, caractérisée en ce qu'elle comporte l'oligomère

AXAXCAAAXAXXAGXGAAGX

avec X = T ou U
ou son complémentaire.

**15.** Sonde d'acides nucléiques spécifique du sous-groupe de virus constitué par HPV5 et HPV8, caractérisée en ce qu'elle comporte l'oligomère

CAAACAACAGXXGCXGACAAXAXXXXAAAAXAXGGCAGXGCXGGXGXAXXX

XXXGG

avec X = T ou U
ou son complémentaire
ou un fragment de ceux-ci comportant entre 18 et 35 oligomères tel que

XAXGGCAGXGCXGGXGXAXXXXXXG

avec X = T ou U
ou son complémentaire.

**16.** Sonde d'acides nucléiques selon l'une des revendications précédentes, caractérisée en ce qu'elle est marquée par l'un des procédés connus pour le marquage des sondes ADN ou ARN, choisi notamment parmi les marquages radioactifs, tels que $^{32}$P et les marquages enzymatiques, tels qu'avec la biotine.

**17.** Procédé de détection d'un type de virus HPV ou d'un sous-groupe de virus HPV à l'aide d'une sonde, selon l'une des revendications précédentes, caractérisé en ce qu'on hybride ladite sonde à une séquence d'ADN ou d'ARN desdits virus HPV, le cas échéant, préalablement dénaturé s'il était initialement bicaténaire.

**18.** Procédé de détection selon la revendication 17, caractérisé en ce que pour les sondes destinées à la détection des gènes E7 des divers virus, on utilise la méthode d'amplification PCR, les cibles identifiées par les sondes étant préalablement amplifiées au moyen des amorces oligonucléotidiques les encadrant suivantes :

|  | position | longueur du fragment amplifié |
|---|---|---|
| pour HPV1a | | |
| GTTGCTTCCTGTGCCTATTGCG | 673-694 | |
| CAGAAGGAGTTCCTCCAGCTG | 762-742 | 90 |
| pour HPV5 | | |
| ATTATTCTGGAGCTCAGTGAGGT | 693-715 | |
| ACAGTCAGGGCACAGGAGCTGC | 944-922 | 252 |
| pour HPV6B | | |
| TGTATTAGACCTGCAACCTCCAG | 562-584 | |
| TTCCCAACAGAAGCTGTTGCAC | 785-764 | 224 |
| pour HPV8 | | |
| CTAGACATCGAAAGAACTGTATTC | 779-802 | |
| GCACTCAGGACACAGAAGCTGT | 934-913 | 156 |

| pour HPV11 | | |
|---|---|---|
| AGTACTAGACCTGCAGCCTCCT | 562-583 | |
| GTAGTTGTCTGATGTCTCCGTC | 767-746 | 206 |
| pour HPV16 | | |
| GCAGAACCGGACAGAGCCCA | 694-713 | |
| GTGTGCCCATTAACAGGTCTTCC | 820-798 | 127 |
| pour HPV18 | | |
| GCCCGACGAGCCGAACCACA | 740-759 | |
| GGAATGCTCGAAGGTCGTCTG | 848-828 | 109 |
| pour HPV33 | | |
| GGCTTGGACCGGCCAGATGG | 681-700 | |
| GTGCACAGGTAGGGCACACAA | 858-838 | 178 |

**Claims**

1. Nucleic acids probe useful for detecting indifferently the various types of human papilloma virus, particularly HPV1a, HPV5, HPV6b, HPV8, HPV11, HPV16, HPV18 and HPV33, consisting of a sequence of labelled nucleic acids, said probe comprising the oligomer of twelve nucleotides X-A-A-A-A-C-G-A-A-A-G-X, with X = T or U, or its complement.

2. Mixture of six nucleic acids probes common to the different types of HPV virus consisting in the mixture of six oligomers, which include all the common sequence of twelve nucleotides of Claim 1, extended by the corresponding nucleotides for each virus type.

3. Mixture of six nucleic probes according to Claim 2, wherein common sequence of twelve nucleotides is extended on each side by 1 to 10 corresponding nucleotides for each of the eight virus types.

4. Mixture of probes according to Claim 3, comprising the mixture of the six following oligomers or their complements :

```
for HPV1a A-X-G-C-X-X-X-A-A-A-A-C-G-A-A-A-G-X-X-A-C-X-X-X

for HPV6b A-G-G-A-C-C-X-A-A-A-A-C-G-A-A-A-G-X-A-X-X-X-A-G
and HPV11

for HPV8 C-A-A-A-A-A-C-X-A-A-A-A-C-G-A-A-A-G-X-A-X-C-X-X-A
and HPV5

for HPV16 A-G-G-X-X-C-X-A-A-A-A-C-G-A-A-A-G-X-A-X-X-X-G-G

for HPV18 A-X-G-X-X-X-X-A-A-A-A-C-G-A-A-A-G-X-X-X-G-C-A-G

for HPV33 G-X-G-C-A-C-X-A-A-A-A-C-G-A-A-A-G-X-X-X-G-C-C-G
```

with X = T or U.

5. Specific probe of nucleic acids for the detection of the HPV1a type of human papilloma virus, said probe comprising an oligomer selected from among :

```
CCXCAGGCGAGAAGCGCGGAC
CXGXCCCXXCXGCGCGAGACGX
XGXCCCXXCXGCGCGAGACGXXGGAAGXAXACACA
```

```
GACCGXCCXCGCGGAXCACAG
GACCGXCCXCGCGGAXCACAGCGCCAXXAGACAGC
```

with X = T or U
and their complements.

6. Specific nucleic acid probe for the detection of the HPV5 type of human papilloma virus, said probe comprising an oligomer selected from among :

XGXAXGGGAXCGGGGGCGXXXA

XXGXAXGGGAXCGGGGGCGXXXA

XGXAXGGGAXCGGGGGCGXXXAGCCAXGGACCAXA

GCCXGACAACGAAAGGAXCXCXXA

GAGGAGCCXGACAACGAAAGGAXCXCXXACAAAGX

with X = T or U
and their complements.

7. Specific probe of nucleic acids for the detection of the HPV6b type of human papilloma virus, said probe comprising an oligomer selected from among :

CAXXAACGCAGGGGCGCCXGAA

GGCAAXCAXXAACGCAGGGGCG

GCAAXCAXXAACGCAGGGGCGCCXGAAAXXGXGCC

GACGAAGXGGACGGACAAGAXXC

GAGGXGGACGAAGXGGACGGACAAGAXXCACAACC

with X = T or U
and their complements.

8. Specific probe of nucleic acids for the detection of the HPV8 type of human papilloma virus, said probe comprising an oligomer selected from among :

ACACCGCCGCCAAGACCCCCA

GCAGCXCAGXCCGCGGCXCC

GCAGCXCAGXCCGCGGCXCCAGXCAAXAXCACXGX

XGXCAACGCAACXGAXXCGGGXAX

CXXXXXGXCAACGCAACXGAXXCGGGXAXCAGGAC

with X = T or U
and their complements.

9. Specific probe of nucleic acids for the detection of the HPV11 type of human papilloma virus, said probe comprising an oligomer selected from among :

GGCGXGXCGGCGCCGCCXAG

GAAGCXCXCCCAAGCCXGCXAXX

XAXAXACCCXXGGGAAGCXCXCCCAAGCCXGCXAX

XGAGGXGGACAAGGXGGACAAAC

AGAAGAXGAGGXGGACAAGGXGGACAAACAAGACG

with X = T or U
and their complements.

10. Specific probe of nucleic acids for the detection of the HPV16 type of human papilloma virus, said probe comprising an oligomer selected from among :

CAGAACCGGACAGAGCCCAX

ACCAGAGACAACXGAXCXCXACXGX

and ACAAGCAGAACCGGACAGAGCCCA

XGXXAGAXXXGCAACCAGAGACAACXGAXCXCXAC

AGXGXGACXCXACGCXXCGGXXG

XXGCAAGXGXGACXCXACGCXXCGGXXGXGCGXAC

with X = T or U
and their complements.

11. Specific probe of nucleic acids for the detection of the HPV18 type of human papilloma virus, said probe comprising an oligomer selected from among :

CGGXAXCCGCXACXCAGCXXG

CCCAXXGXAXCACCCACGGCCC

CCCAXXGXAXCACCCACGGCCCCXGCCXCXACACA

XXGXAAGXGXGAAGCCAGAAXXGAG

AXGXGXXGXAAGXGXGAAGCCAGAAXXGAGCXAGX

with X = T or U
and their complements.

12. Specific probe of nucleic acids for the detection of the HPV33 type of human papilloma virus, said probe comprising an oligomer selected from among :

CGXCCXCCGGXXACXGXAGAXA

GXCCXCCGGXXACXGXAGACACX

XCCXCCGGXXACXGXAGACACXGXXGGACCXXXAG

AGCAAGXGACCXACGAACCAXACA

AGXACAGCAAGXGACCXACGAACCAXACAGCAACX

with X = T or U
and their complements.

13. Specific probe of nucleic acids of the sub-group of HPV virus constituted by HPV16, HPV18 and HPV33, said probe comprising the oligomer
XXAGGCACAXAXXXX
with X = T or U
or its complement.

14. Specific probe of nucleic acids of the sub-group of HPV virus constituted by HPV16 and HPB18, said probe comprising the oligomer
AXAXCAAAXAXXAGXGAAGX
with X = T or U
or its complement.

15. Specific probe of nucleic acids of the sub-group of HPV virus constituted by HPV5 and HPV8, said probe comprising the oligomer
CAAACAACAGXXGCXGACAAXAXXXXAAAAXAXGGCAGXGCXGGXGXAXXXXXXGG
   with X = T or U
or its complement
or a fragment of the latter comprising between 18 and 35 oligomers such as
XAXGGCAGXGCXGGXGXAXXXXXXG
   with X = T or U
or its complement.

16. Probe of nucleic acids according to one of the preceding Claims, said probe being labelled by one of the methods known for the labelling of DNA or RNA probes, selected particularly from among radioactive labelling, such as 32p and enzymatic labelling, such as with biotin.

17. Method for detecting a type of HPV virus or of a sub-group of HPV virus by means of a probe, according to one of the preceding Claims, said method comprising hybridizing said probe with a DNA or RNA sequence of said HPV virus, as the case may be, previously denatured if it was initially bicatenary.

18. Method of detection according to Claim 17, comprising using the PCR method of amplification with following oligomeric primers :

|  | position | length of amplified fragment |
|---|---|---|
| for HPV1a | | |
| GTTGCTTCCTGTGCCTATTGCG | 673-694 | |
| CAGAAGGAGTTCCTCCAGCTG | 762-742 | 90 |
| for HPV5 | | |
| ATTATTCTGGAGCTCAGTGAGGT | 693-715 | |
| ACAGTCAGGGCACAGGAGCTGC | 944-922 | 252 |
| for HPV6B | | |
| TGTATTAGACCTGCAACCTCCAG | 562-584 | |
| TTCCCAACAGAAGCTGTTGCAC | 785-764 | 224 |
| for HPV8 | | |
| CTAGACATCGAAAGAACTGTATTC | 779-802 | |
| GCACTCAGGACACAGAAGCTGT | 934-913 | 156 |
| for HPV11 | | |
| AGTACTAGACCTGCAGCCTCCT | 562-583 | |
| GTAGTTGTCTGATGTCTCCGTC | 767-746 | 206 |
| for HPV16 | | |
| GCAGAACCGGACAGAGCCCA | 694-713 | |
| GTGTGCCCATTAACAGGTCTTCC | 820-798 | 127 |

```
for  HPV18

GCCCGACGAGCCGAACCACA          740-759
                                              109
GGAATGCTCGAAGGTCGTCTG         848-828

for HPV33

GGCTTGGACCGGCCAGATGG          681-700
                                              178
GTGCACAGGTAGGGCACACAA         858-838
```

**Patentansprüche**

1. Nucleinsäuren-Sonde für den reaktionslosen Nachweis verschiedener Typen des Human-Papillom-Virus, insbesondere von HPV1a, HPV5, HPV6b, HPV8, HPV11, HPV16, HPV18 und HPV33, die aus einer markierten Nucleinsäure-Sequenz besteht, dadurch gekennzeichnet, daß sie umfaßt ein Oligomer von zwölf Nucleotiden der Formel,
X-A-A-A-A-C-G-A-A-A-G-X,
worin X = T oder U, oder sein Komplement.

2. Gemisch von sechs Nucleinsäuren-Sonden, die unterschiedlichen Typen des HPV-Virus gemeinsam sind, das besteht aus einem Gemisch von sechs Oligomeren, die alle die gemeinsame Sequenz von zwölf Nucleotiden gemäß Anspruch 1 aufweisen, verlängert durch die entsprechenden Nucleotide für jeden Virus-Typ.

3. Gemisch von sechs Nucleinsäuren-Sonden nach Anspruch 2, dadurch gekennzeichnet, daß die gemeinsame Sequenz von zwölf Nucleotiden für jeden der acht Virus-Typen beiderseits durch 1 bis 10 entsprechende Nucleotide verlängert ist.

4. Gemisch von Sonden nach Anspruch 3, dadurch gekennzeichnet, daß es umfaßt ein Gemisch der folgenden sechs Oligomeren oder ihrer Komplemente:

```
für  HPV1a     A-X-G-C-X-X-X-A-A-A-A-C-G-A-A-AG-X-X-A-C-X-X-X

für  HPV6b     A-G-GA-C-C-X-A-A-A-A-C-G-A-A-A-G-X-A-X-X-X-A-G
und HPV11

für  HPV8      -A-A-A-A-A-C-X-A-A-A-A-C-G-A-A-A-G-X-A-X-C-X-X-A
und HPV5

für  HPV16     A-G-G-X-X-C-X-A-A-A-A-C-G-A-A-A-G-X-A-X-X-X-G-G

für  HPV18     A-X-G-X-X-X-X-A-A-A-A-C-G-A-A-A-G-X-X-X-G-C-A-G

für  HPV33     G-X-G-C-A-C-X-A-A-A-A-C-G-A-A-A-G-X-X-X-G-C-C-G
```

worin X = T oder U.

5. Nucleinsäuren-Sonde, die spezifisch ist für den Nachweis des Human-Papillom-Virus-Typs HPV1a, dadurch gekennzeichnet, daß sie umfaßt ein Oligomer, ausgewählt aus:

CCXCAGGCGAGAAGCGCGGAC

CXGXCCCXXCXGCGCGAGACGX

XGXCCCXXCXGCGCGAGACGXXGGAAGXAXACACA

GACCGXCCXCGCGGAXCACAG

GACCGXCCXCGCGGAXCACAGCGCCAXXAGACAGC

worin X = T oder U, und ihren Komplementen.

6. Nucleinsäuren-Sonde, die spezifisch ist für den Nachweis des Human-Papillom-Virus-Typs HPV5, dadurch gekennzeichnet, daß sie umfaßt ein Oligomer, ausgewählt aus:

XGXAXGGGAXCGGGGGCGXXXA

XXGXAXGGGAXCGGGGGCGXXXA

XGXAXGGGAXCGGGGGCGXXXAGCCAXGGACCAXA

GCCXGACAACGAAAGGAXCXCXXA

GAGGAGCCXGACAACGAAAGGAXCXCXXACAAAGX

worin X = T oder U, und ihren Komplementen.

7. Nucleinsäuren-Sonde, die spezifisch ist für den Nachweis des Human-Papillom-Virus-Typs HPV6b, dadurch gekennzeichnet, daß sie umfaßt ein Oligomer, ausgewählt aus:

CAXXAACGCAGGGGCGCCXGAA

GGCAAXCAXXAACGCAGGGGCG

GCAAXCAXXAACGCAGGGGCGCCXGAAAXXGXGCC

GACGAAGXGGACGGACAAGAXXC

GAGGXGGACGAAGXGGACGGACAAGAXXCACAACC

worin X = T oder U, und ihren Komplementen.

8. Nucleinsäuren-Sonde, die spezifisch ist für den Nachweis des Human-Papillom-Virus-Typs HPV8, dadurch gekennzeichnet, daß sie umaßt ein Oligomer, ausgewählt aus:

ACACCGCCGCCAAGACCCCCA

GCAGCXCAGXCCGCGGCXCC

GCAGCXCAGXCCGCGGCXCCAGXCAAXAXCACXGX

XGXCAACGCAACXGAXXCGGGXAX

CXXXXXGXCAACGCAACXGAXXCGGGXAXCAGGAC

worin X = T, oder U und ihren Komplementen.

9. Nucleinsäuren-Sonde, die spezifisch ist für den Nachweis des Human-Papillom-Virus-Typs HPV11, dadurch gekennzeichnet, daß sie umfaßt ein Oligomer, ausgewählt aus:

GGCGXGXCGGCGCCGCCXAG

GAAGCXCXCCCAAGCCXGCXAXX

XAXAXACCCXXGGGAAGCXCXCCCAAGCCXGCXAX

XGAGGXGGACAAGGXGGACAAAC

AGAAGAXGAGGXGGACAAGGXGGACAAACAAGACG

worin X = T oder U, und ihren Komplementen.

**10.** Nucleinsäuren-Sonde, die spezifisch ist für den Nachweis des Human-Papillom-Virus-Typs HPV16, dadurch gekennzeichnet, daß sie umfaßt ein Oligomer, ausgewählt aus:

CAGAACCGGACAGAGCCCAX

ACCAGAGACAACXGAXCXCXACXGX

und: ACAAGCAGAACCGGACAGAGCCCA

XGXXAGAXXXGCAACCAGAGACAACXGAXCXCXAC

AGXGXGACXCXACGCXXCGGXXG

XXGCAAGXGXGACXCXACGCXXCGGXXGXGCGXAC

worin X = T oder U, und ihren Komplementen.

**11.** Nucleinsäuren-Sonde, die spezifisch ist für den Nachweis des Human-Papillom-Virus-Typs HPV18, dadurch gekennzeichnet, daß sie umfaßt ein Oligomer, ausgewählt aus:

CGGXAXCCGCXACXCAGCXXG

CCCAXXGXAXCACCCACGGCCC

CCCAXXGXAXCACCCACGGCCCCXGCCXCXACACA

XXGXAAGXGXGAAGCCAGAAXXGAG

AXGXGXXGXAAGXGXGAAGCCAGAAXXGAGCXAGX

worin X = T oder U, und ihren Komplementen.

**12.** Nucleinsäuren-Sonde, die spezifisch ist für den Nachweis des Human-Papillom-Virus-Typs HPV33, dadurch gekennzeichnet, daß sie umfaßt ein Oligomer, ausgewählt aus:

CGXCCXCCGGXXACXGXAGAXA

GXCCXCCGGXXACXGXAGACACX

XCCXCCGGXXACXGXAGACACXGXXGGACCXXXAG

AGCAAGXGACCXACGAACCAXACA

AGXACAGCAAGXGACCXACGAACCAXACAGCAACX

worin X = T oder U, und ihren Komplementen.

**13.** Nucleinsäuren-Sonde, die spezifisch ist für die Untergruppe des HPV-Virus, bestehend aus HPV16, HPV18 und HPV33, dadurch gekennzeichnet, daß sie umfaßt das Oligomer XXAGGCACAXAXXXX
worin X = T oder U, oder sein Komplement.

29

14. Nucleinsäuren-Sonde, die spezifisch ist für die Untergruppe des Virus, bestehend aus HPV16 und HPV18, dadurch gekennzeichnet, daß sie umfaßt das Oligomer
AXAXCAAAXAXXAGXGAAGX
worin X = T oder U, oder sein Komplement.

15. Nucleinsäuren-Sonde, die spezifisch ist für die Untergruppe des Virus, bestehend aus HPV5 und HPV8, dadurch gekennzeichnet, daß sie umfaßt das Oligomer

CAAACAACAGXXGCXGACAAXAXXXXAAAAXAXGGCAGXGCXGGXGXAXXX
XXXGG

worin X = T oder U, oder sein Komplement
oder ein Fragment davon, das umfaßt zwischen 18 und 35 Oligomere, wie
XAXGGCAGXGCXGGXGXAXXXXXXG
worin X = T oder U, oder sein Komplement.

16. Nucleinsäuren-Sonde nach einem der vorhergehenden Ansprüche, dadurch gekennzeichnet, daß sie markiert ist nach einem der für die Markierung der DNA- oder RNA-Sonden bekannten Verfahren, ausgewählt insbesondere aus den radioaktiven Markierungen wie $^{32}$P und den enzymatischen Markierungen, wie Biotin.

17. Verfahren zum Nachweis eines Typs des HPV-Virus oder einer Untergruppe des HPV-Virus mit Hilfe einer Sonde nach einem der vorhergehenden Ansprüche, dadurch gekennzeichnet, daß man die genannte Sonde mit einer DNA- oder RNA-Sequenz des genannten HPV-Virus hybridisiert, die gegebenenfalls vorher denaturiert worden ist, wenn sie anfänglich zweisträngig war.

18. Nachweisverfahren nach Anspruch 17, dadurch gekennzeichnet, daß man als Sonden, die zum Nachweis der E7-Gene verschiedener Viren bestimmt sind, die PCR-Amplifikations-Methode anwendet, bei der die durch die Sonden identifizierten Ziele vorher amplifiziert worden sind mittels der folgenden, sie inserierenden Oligonucleotid-Aktivatoren:

|  | Position | Länge des ampli-<br>fizierten Fragments |
|---|---|---|

für HPV1a

GTTGCTTCCTGTGCCTATTGCG    673-694

CAGAAGGAGTTCCTCCAGCTG    762-742

                                                       90

für HPV5

ATTATTCTGGAGCTCAGTGAGGT    693-715

ACAGTCAGGGCACAGGAGCTGC    944-922

                                                    252

für HPV6B

TGTATTAGACCTGCAACCTCCAG    562-584

TTCCCAACAGAAGCTGTTGCAC    785-764

                                                  224

für HPV8

CTAGACATCGAAAGAACTGTATTC   779-802

GCACTCAGGACACAGAAGCTGT    934-913

                                                  156

für HPV11

AGTACTAGACCTGCAGCCTCCT    562-583

GTAGTTGTCTGATGTCTCCGTC    767-746

                                                  206

für HPV16

GCAGAACCGGACAGAGCCCA    694-713

GTGTGCCCATTAACAGGTCTTCC   820-798

                                                  127

für HPV18

GCCCGACGAGCCGAACCACA    740-759

GGAATGCTCGAAGGTCGTCTG    848-828

                                                  109

für HPV33

GGCTTGGACCGGCCAGATGG    681-700

GTGCACAGGTAGGGCACACAA    858-838

                                                  178

FIG_1

FIG-2

FIG.3

FIG.4

FIG.5